# EUROPEAN PATENT SPECIFICATION

(11) **EP 4 137 039 B1**
(45) Date of publication and mention of the grant of the patent: **27.03.2024**
(21) Application number: 21306130.2
(22) Date of filing: 20.08.2021
(51) Int. Cl.: A61B 5/00, H01R 12/71

(54) **CONNECTION TRANSMITTER WITH CONNECTION BLADES**
VERBINDUNGSSENDER MIT VERBINDUNGSBLÄTTERN
ÉMETTEUR DE CONNEXION AVEC LAMES DE CONNEXION

(43) Date of publication of application: 22.02.2023
(73) Proprietor: Sensome, 91300 Massy (FR)
(72) Inventor: LECOURT, Guillaume, 91300 Massy (FR); LEBEDEV, Gor, 91300 Massy (FR); CARREEL, Bruno, 94110 Arcueil (FR)
(74) Representative: Icosa

(56) References cited:
- US-A1- 2004 186 542
- US-A1- 2010 273 355
- US-A1- 2014 180 139

## Description

### FIELD OF INVENTION

The present invention relates to safely connecting a guidewire to a connector, for example a circuit board assembly.

### BACKGROUND OF INVENTION

Guidewire assemblies comprising guidewire with a sensor mounted at its distal tip are routinely used in intravascular imaging. The sensor of the distal tip of the guidewire is usually connected to an interface cable, connecting to external equipment such as for example monitors, control units or computers.

Blockages of blood vessels (including veins or arteries) may occur in various parts of an animal (e.g., a human or a non-human animal) and may have significant repercussions. In an ischemic stroke, for example, a blood clot fully or partially blocks blood flow in a cerebral artery. If the clot is not treated quickly, insufficient blood flow may cause irreparable damage to the brain.

Blockages may be caused by blood clots, which may be caused by coagulation of red and/or white blood cells and/or platelets within a blood vessel. The coagulation may be triggered by a variety of factors, including an injury, abnormal blood flow at the site of the blockage, a disease/condition predisposing an animal to coagulation, and/or other factors.

A common treatment of a clot is chemical dissolution of the clot, which is feasible within the first 4.5 hours following blockage of a blood vessel. Another common option is mechanical thrombectomy, in which an aspiration catheter or a stent-retriever is used to remove the blood clot from the blood vessel.

Stent-retrievers include a stent attached at the end of a wire. The stent is deployed into the vasculature and into the clot, expanded into the clot, and, after a typical waiting time of 0.5 to 10 minutes, extracted to pull the clot out of the blood vessel. Due to non-optimal grabbing of the clot by the stent-retriever, some parts of the clot may be left by or be lost from the retriever, such that several succession treatments (an average 3 times) may be necessary to treat the blockage and restore circulation in the vessel.

An early determination of the nature of the clot, performed using a guidewire as describe above, may result in an enhanced treatment of said clot. A known technique for determining the nature of the clot includes applying the sensor assembly of the guidewire to the clot, in order to measure an evolution of the clot impedance over a predetermined frequency range. The corresponding electrical signals are conveyed along the length of the guidewire, which is connected, at its proximal end, to a processing unit, through a connection transmitter.

Document US 6,428,336 discloses a known connection transmitter. This transmitter comprises a series of electrical contacts arranged along a longitudinal axis. The proximal end of the guidewire is inserted along said longitudinal axis, until each electrical contact of the connection transmitter is in electrical contact with a corresponding guidewire contact, said guidewire contacts being successively arranged along a longitudinal direction of the guidewire, at a distance from each other.

Document US 2014/180139 discloses side-loading electrical connectors for use with intravascular devices. The side-loading electrical connector has at least one electrical contact configured to interface with an electrical connector of the intravascular device. A first connection piece of the side-loading electrical connector is movable relative to a second connection piece between an open position and a closed position, wherein in the open position an elongated opening is formed between the first and second connection pieces to facilitate insertion of the electrical connector between the first and second connection pieces in a direction transverse to a longitudinal axis of the intravascular device and wherein in the closed position the at least one electrical contact is electrically coupled to the at least one electrical connector received between the first and second connection pieces.

However, such device is not satisfactory. Indeed, the operation described above results in greater wear of the guidewire contacts that are closer to the tip of the guidewire, due to the successive friction of the guidewire contacts against the electrical contacts of the connection transmitter. Moreover, handling of the guidewire and the connection transmitter during said connection is cumbersome, for instance due to said friction, which may result in breakage of the guidewire.

Therefore, a purpose of the invention is to provide a connection transmitter that is easier to use than the known connection transmitters, while reducing the risks of deterioration of the guidewire.

### SUMMARY

This invention thus relates to a connection transmitter aimed at connecting a guidewire to a connector assembly, the connection transmitter comprising:
- a connector assembly,
- a casing surrounding at least partially the connector assembly,
- a longitudinal connecting element extending inside the casing along a longitudinal axis,

**wherein** the connecting element is translatable, within the casing, along a transversal axis perpendicular to the longitudinal axis, said longitudinal connecting element being further aimed at receiving the guidewire,
**wherein** the connector assembly comprises at least one connection blade extending along the transversal axis, and the connecting element comprises at least one transversal cavity aimed at receiving at least partially the guidewire, and further aimed at cooperating, by transversal translation, with the at least one connection blade,
**wherein** the connection transmitter presents at least two configurations:
   - a non-connecting configuration, in which the at least one transversal cavity of the longitudinal connecting element and the at least one connection blade of the connector assembly are transversely disengaged,
   - a connecting configuration, in which the at least one transversal cavity of the longitudinal connecting element and the at least one connection blade of the connector assembly are transversely cooperating by engagement,
**wherein** the connecting configuration enables to connect the guidewire introduced inside the longitudinal connecting element with the at least connection blade of the connector assembly.

Thus, this solution achieves the above objective. In particular, it allows the obtaining of a reduction of the wearing of the connection blades and further of a reduced homogeneous wear of said blade while providing an easy and handy connection device.

The connection transmitter according to the invention may include one or more of the following characteristics, taken in isolation from one another or in combination with one another:
- the longitudinal connecting element may comprise a longitudinal cavity extending along the longitudinal axis, said longitudinal cavity being aimed at receiving the guidewire, said longitudinal cavity intersecting each transversal cavity of the longitudinal connecting element,
- the longitudinal connecting element may comprise at least one radial stop configured to prevent radial or transversal motion of the guidewire once said guidewire is introduced in the longitudinal connecting element, only allowing the longitudinal sliding of said guidewire,
- the connecting configuration of the connection transmitter may be obtained by translating the longitudinal connecting element along the transversal axis, thus enabling to connect the guidewire introduced inside the longitudinal connecting element with each connection blade of the connector assembly at the same time,
- the number of connection blades may be equal to the number of transversal cavities such that each connection blade corresponds to a respective transversal cavity,
- the default configuration of the connection transmitter may be the connecting configuration, which is maintained by means of transversal locking element aimed at transversally cooperating with the casing,
- the transversal locking element may comprise a spring aimed at extending between the casing and the longitudinal connecting element,
- the longitudinal connecting element and the casing may both comprise wire longitudinal locking elements aimed at cooperating together when the connection transmitter is in its connecting configuration in order to prevent longitudinal sliding of the introduced guidewire,
- the longitudinal connecting element may comprise an activation switch to switch the longitudinal connecting element between the connecting configuration and the non-connecting configuration, said activation switch being operable from outside the casing,
- the activation switch may comprise a slider aimed at being pushed with a user's thumb.

The invention further relates to a connection kit comprising a connection transmitter according to any one of the hare above listed technical features, and a guidewire aimed at being introduced at least partially inside the connection transmitter.

The connection kit according to the invention may include one or more of the following characteristics, taken in isolation from one another or in combination with one another:
- the connector assembly may comprise N connection blades extending along the transversal axis, and wherein the longitudinal connecting element comprises N transversal cavities, each transversal cavity being aimed at cooperating, by transversal translation, with one corresponding connection blade of the connector assembly, N being an integer equal to a number of contact zones on a proximal end of the guide wire,
- a first end of the guidewire may comprise a series of contact zones arranged along a longitudinal direction of the guidewire, each electrical contact being associated to a respective transversal cavity, a distance along the longitudinal axis between two transversal cavities being equal to a distance, along the longitudinal axis of the guidewire, between the two corresponding contact zones.

The invention further relates to a connection process aimed at connecting a guidewire to a connector assembly of a connection transmitter, the connection process being implemented by means of a connection transmitter according to any one the hereabove listed technical features, said connection process comprising following steps in the order of enunciation:
- longitudinally introducing a first end of the guidewire inside the longitudinal connecting element,
- switching the connection transmitter into its connecting configuration, in order to put the guidewire in contact with at least one of the connection blades of the connector assembly.

The process according to the present invention may comprise a step before the introduction of the guidewire inside the longitudinal connecting element, said additional step being the switching of the connection transmitter into its non-connecting configuration.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings:
- **Figure 1** is a general perspective view of the present invention,
- **Figure 2** is an exploded view of the present invention the longitudinal connecting element being outside the casing,
- **Figure 3** is an exploded view of the present invention the longitudinal connecting element being inside the casing,
- **Figure 4** is an inside perspective view of the present invention,
- **Figures 5a, 5b****,** are two different views of the longitudinal connecting element of the present invention, respectively a perspective view and a sectional side view,
- **Figure 5c** is a sectional transverse view of a slider according to the present invention,
- **Figures 6** is a perspective view of a connector assembly according to the invention,
- **Figures 7a, 7b, 7c** is a three steps view of the process of connecting a guidewire to a connector assembly by means of the present invention,
- **Figure 8** is a sectional side view of a guidewire connected to a connector assembly by means of the present invention.

### DETAILED DESCRIPTION

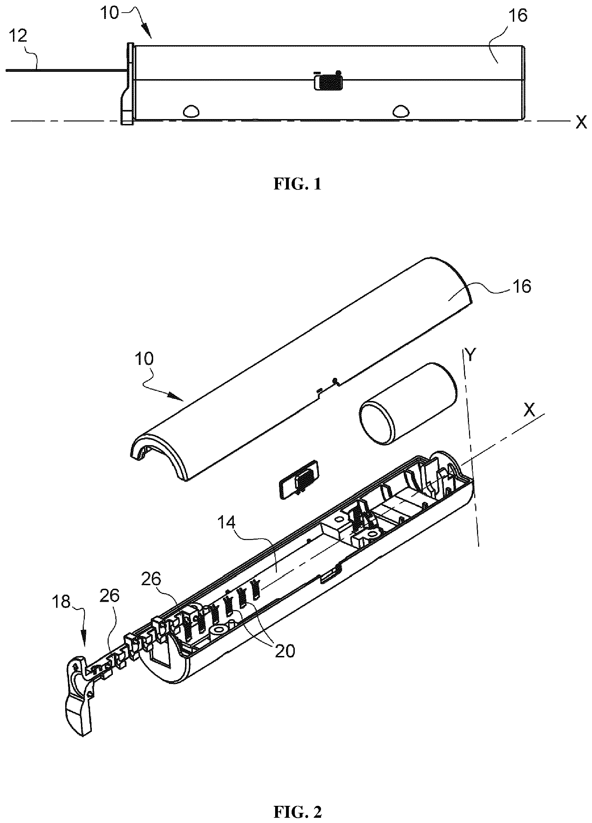

As can be seen on figure 1, the present invention relates to a connection transmitter 10 aimed at connecting a guidewire 12 to a connector assembly 14, for example a printed circuit board assembly. The connection transmitter 10 is aimed at being handled by a health professional during operation of the guidewire 12.

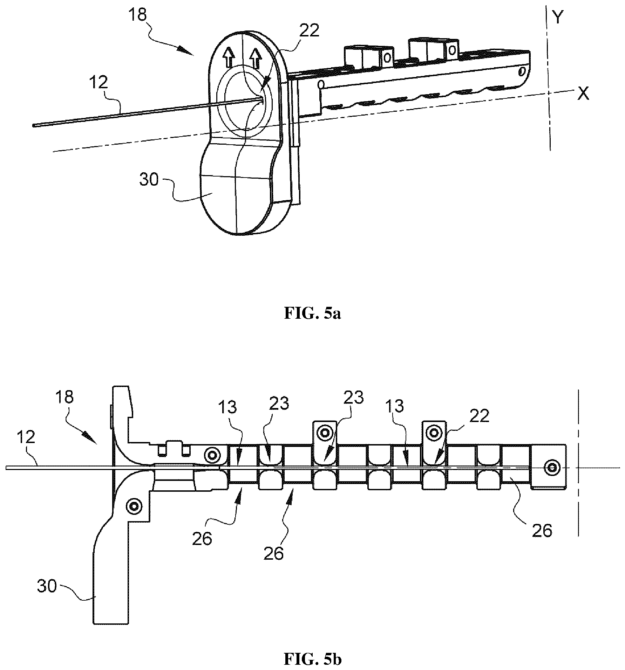

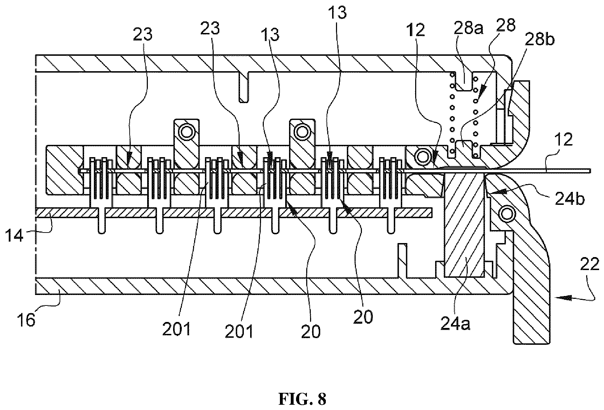

As well known by any person skilled in the art, a guidewire 12 is a wire or spring used as a guide for placement of a larger device or prosthesis, such as a catheter or intramedullary pin, inside a patient's body. The guidewire may also be used to probe tissues of a patient, such as tissues surrounding a clot or the clot itself. The present invention is about connecting a guidewire 12 of about 0,2 to 0,5mm, preferably 0,35mm. In this particular intention, the guidewire 12 comprises at least one circumferential contact zone 13 (see figures 5b and 8) arranged along a longitudinal direction of the guidewire. Those contact zones are aimed at establishing a connection with the connector assembly 14.

As shown on the figures, the connection transmitter 10 according to the present invention comprises:
- a connector assembly 14, in this case a printed circuit board assembly,
- a casing 16 which preferably extends along a longitudinal axis X,
- a longitudinal connecting element 18 extending inside the casing 16 along the longitudinal axis X.

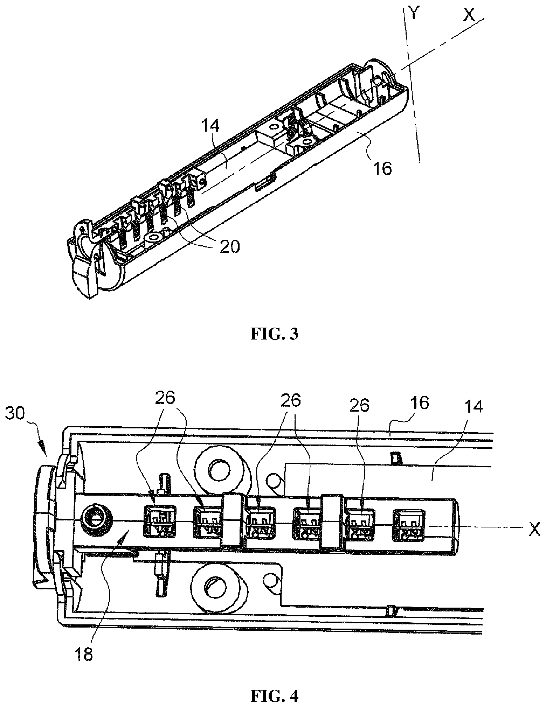

As can be seen on figures, 2, 3 and 4, the casing 16 surrounds at least partially the connector assembly 14. The casing displays a general cylindric shape, as can be seen on figure 1. In the embodiment represented on figures 2 and 3, the casing 16 comprises two parts fixed together, for instance screwed together around the connector assembly 14. The casing 16 displays one opening 16a at one of its longitudinal extremities, said opening 16a receiving the longitudinal connecting element 18.

In some preferred embodiments, the material used for the casing 16 comprises a resin manufactured by FormLabs^{®}: White Resin Standard. It is an ABS-like resin usually used for stereolithography printing. Regarding the size, the casing 16 presents a length comprised between 140 and 150 mm, preferably 148.5 mm and a diameter comprised between 25 and 30 mm, preferably 28 mm.

In some embodiments, the casing 16 is a removable casing.

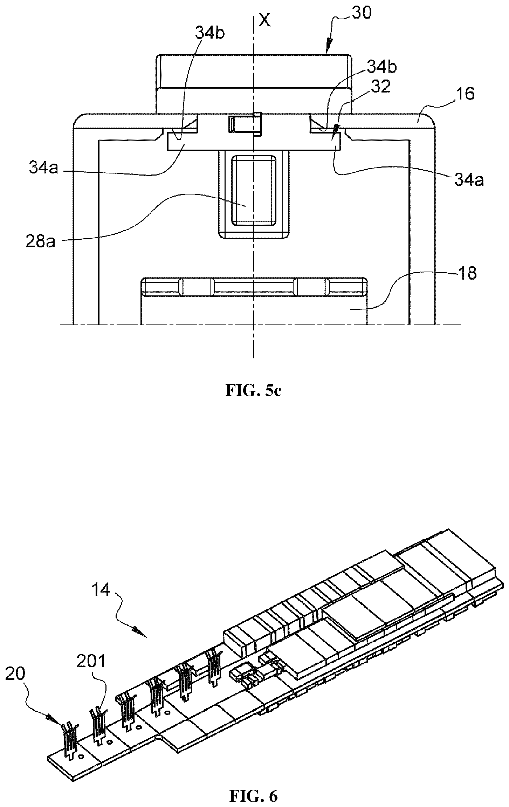

In some embodiments, the connector assembly may be, as illustrated in figure 6, a board assembly, and more precisely a printed circuit board assembly (PCBA). Regardless of the embodiment, the size of the connector assembly 14 is dependent on the components used to be functional. Similarly, the shape of the connector assembly 14 is designed according to the guidewire 12 to be connected.

The connector assembly 14 comprises at least one connection blade 20 extending along a transversal axis Y. Each blade 20 is aimed at being put in contact with one corresponding contact zone 13 of the guidewire 12, in order to establish an electrical connection.

The transversal axis Y is sensibly perpendicular to the longitudinal axis X.

Each connecting blade 20 presents a width comprised between 2 and 3 mm, preferably 2,6 mm and a height comprised between 6 and 8 mm, preferably 7,5 mm.

Regarding the present invention, each connection blade 20 comprises at least one finger 201 at its free end. In one embodiment (not represented) the finger may display a general "C" shaped portion either it the extremity of the finger 201 or at its end. The guidewire 12 can cross said C shaped section. In an alternative, not represented embodiment, each finger 201 is simply slightly inclinable and bends under the pressure force exerted by and/or the weight of the guidewire put in contact with it. The finger 201 could also be provided, on its extremity, with a platform aimed at connecting the guidewire 12. On the embodiment illustrated on figure 6, each connection blade 20 displays a general comb shape with four fingers 201. More precisely, in this embodiment, the free end of each finger 201 presents a bending in one or the other radial direction. The bending of the fingers 201 alternates along the longitudinally X axis. Those bent fingers 201 thus generate a general V shape of the free ends of each blade 20 which creates a funnel for an introduced guidewire 12.

The blades 20 preferably comprise some kind of copper-beryllium alloy (Cu Be) and are further coated with Nickel (Ni) and Gold (Au) to enhance their electrical properties.

Regarding the illustrated embodiment, the connector assembly 14 comprises six connection blades 20 extending along the transversal axis Y. The six connection blades 20 are aligned along the longitudinal X axis, aligned with the longitudinal connecting element 18 (see figures 2 and 3). More generally, the number of connection blades 20 is at least equal to the number of contact zones 13 of the guidewire with which the connection transmitter 10 is meant to cooperate.

The longitudinal connecting element 18 presents a length comprised between 30 and 50 mm, preferably 42 mm.

The longitudinal connecting element 18 is translatable, within the casing 16, through the opening 16a, along the transversal axis Y. The connecting element 18 is longitudinally secured to the casing in order to avoid any unwanted longitudinal sliding along the X axis, as will be later described. When the connection transmitter 10 is functional, the connecting element 18 can only translate transversally along the Y axis.

The longitudinal connecting element 18 is aimed at receiving a guidewire 12. More precisely, as can be seen on figures 5a and 5c, the longitudinal connecting element 18 comprises a longitudinal cavity 22 extending along the longitudinal axis X. In those embodiments, this longitudinal cavity 22 is aimed at receiving the guidewire 12.

The longitudinal connecting element 18 is specifically designed to avoid radial or transversal sliding of the guidewire 12 once it is introduced in it. More precisely, the longitudinal connecting element 18 only allows the longitudinal sliding of the guidewire 12, as can be deduced from figures 5a, 5b, 5c, 7a, 7b, 7c and 8. In order to achieve this, the longitudinal connecting element 18 comprises at least one radial stop 23. In the embodiment depicted on figures 5a, 5b, 5c, 7a, 7b, 7c and 8, the radial stop 23 is the wall of the longitudinal cavity 22 itself. In some alternative not represented embodiment, the radial stop 23 might be a set of internal teeth or spikes or a specific external wall texture.

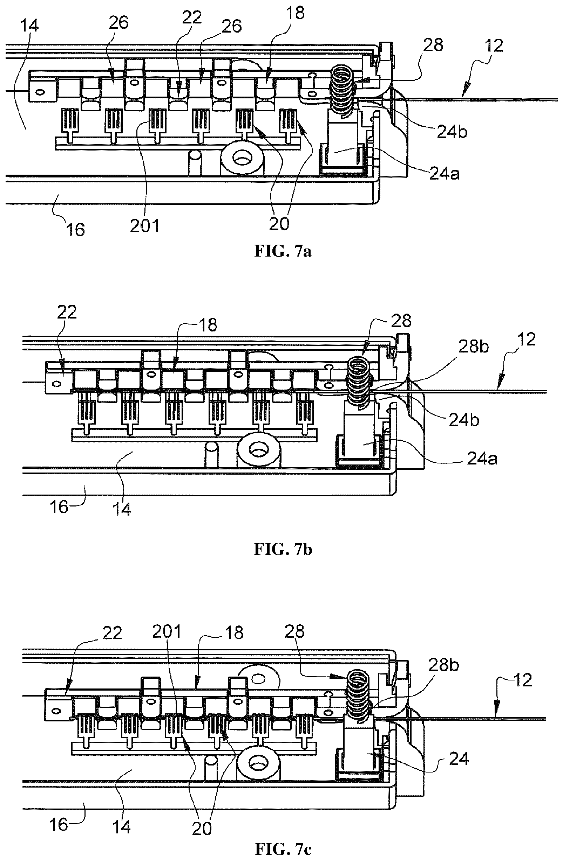

Once the guidewire 12 is introduced inside the longitudinal connecting element, it is important that the guidewire remains in place, in all directions. Therefore, in order to further lock the introduced guidewire 12 longitudinally along the X axis, the longitudinal connecting element 18 and the casing 16 both comprise wire longitudinal locking elements 24 aimed at cooperating together when activated. On the depicted embodiment, as can be seen on figures 7a, 7b, 7c and 8, said wire longitudinal locking means comprise a locking block 24a secured to the casing 16 while the longitudinal connecting element 18 presents a corresponding transversal locking cavity 24b. In some embodiments, locking block 24a can be a soft polymer (PDMS) block. Said transversal locking cavity 24b opens inside the longitudinal cavity 22 of the longitudinal connecting element 18. The transversal locking cavity 24b is stationary with regards to the longitudinal connecting element 18. This way, the transversal locking cavity 24b is preferably transversally mobile with respect to the polymer locking block 24a. Thus, depending on the transversal position along the Y axis of the longitudinal connecting element 18 inside the casing 16, the polymer locking block 24a may be protruding inside the longitudinal cavity 22 of the longitudinal connecting element 18 and obturate it (see figures 7c and 8) or remain outside said longitudinal cavity 22 (see figures 7a, 7b). In its protruding position the polymer locking block 24a may cooperate by friction with the introduced guidewire 12, thus preventing it from sliding along the X axis.

Advantageously, the longitudinal connecting element 18 further comprises a longitudinal registration element, for instance a distal wall of the longitudinal cavity 22. The longitudinal registration element is designed so that, when the distal tip the guidewire 12 rests against said longitudinal registration element, each contact zone 13 of the guidewire 12 is located in a respective transversal cavity 26 of the longitudinal connecting element 18. Said transversal cavities 26 will be described below.

The longitudinal connecting element 18 further comprises at least one transversal cavity 26 extending along the Y axis. This at least one transversal cavity 26 is aimed at cooperating, by transversal translation, with the at least one connection blade 20 of the connector assembly 14. The number of connection blades 20 is preferably the same as the number of transversal cavities 26. This way, each connection blade 20 corresponds to one corresponding transversal cavity 26. For example, on the embodiment illustrated on figure 4, the longitudinal connecting element 18 comprises six transversal cavities 26, each transversal cavity 26 corresponding to a connecting blade 20 with which it is aimed to cooperate. Each cavity presents two openings along the Y axis, on each surface of the longitudinal connecting element 18. In an alternative embodiment, each transversal cavity 26 presents only one opening on the surface facing the connector assembly 14. The transversal cavities are preferably square shaped. The size of each transversal cavity 26 is comprised between 2 and 5mm length or width. In case the transversal cavities 26 are squares, they preferably measure 4mm width and 4mm length, meaning that, when connected to the guidewire 12, each transversal cavity 26 extends 2mm on each side of the guidewire 12, each half transversal cavity 26 having a width of 2mm and a length of 4mm.

As can be seen on figures 7a and 8, the longitudinal cavity 22 of the longitudinal connecting element 18 intersects each of its transversal cavity 26 (see figure 5c). This means that a guidewire 12 introduced into the longitudinal cavity 22 of the longitudinal connecting element 18 may crosse each of the transversal cavities 26 of the longitudinal connecting element 18 as illustrated on figures 5c, 7c and 8.

As can be seen on figure 7a, 7b and 7c, the connection transmitter 10 according to the present invention presents at least two configurations:
- a non-connecting configuration, in which the transversal cavities 26 of the longitudinal connecting element 18 and the connection blades 20 of connector assembly 14 are transversely disengaged (see figure 7a),
- a connecting configuration, in which the transversal cavities 26 of the longitudinal connecting element 18 and each corresponding connection blade 20 of the connector assembly 14 are transversely cooperating by engagement (see figure 7c and 8).

The configuration of the connection transmitter 10 is changed by translating transversally (along the Y axis) the longitudinal connecting element 18 within the casing 16.

In the non-connecting configuration, the transversal cavities 26 of the longitudinal connecting element 18 and the connection blades 20 of the connector assembly 14 are positioned in two distinct planes, with respect to the transversal axis Y.

In the connecting configuration, the transversal cavities 26 of the longitudinal connecting element 18 and the connection blades 20 of the connector assembly 14 are positioned in the same plane, regarding the transversal axis Y, leading to a transversal cooperation by engagement, at least partially. Thus, if a guidewire 12 is introduced inside the longitudinal connecting element 18 in the non-connecting configuration, the, by transitioning into this connecting configuration, the guidewire 12 is put in contact with at least one of the connecting blades 20 of the connector assembly 14, as the blades 20 fill the space within the transversal cavities 26 crossed by the guidewire 12. This way, the connecting configuration enables to connect the guidewire 12 introduced inside the longitudinal connecting element 18, and more specifically the contact zones 13, with the connection blades 20 of the connector assembly 14. This is illustrated on figure 8.

Regarding the wire longitudinal locking elements 24, when the connection transmitter 10 is in its connecting configuration, as explained above, the polymer locking block 24a secures the guidewire 12 thanks to friction. However, in case no guidewire 12 has been introduced inside the longitudinal connecting element 18 yet, if the connection transmitter 10 is in its connecting configuration, the polymer locking block 24a has a further purpose: preventing a user to insert the guidewire 12 without activating the longitudinal connecting element 18 and sliding it into the non-connecting configuration in which the connection blades 20 are disengaged from the transversal cavities 26. This protects the connecting blades 20 that could be damaged if a lateral stress is applied to them by forcing a wire among them longitudinally (*i*.*e*., along the longitudinal axis X) instead of transversally.

One of the main issues of any kind of connection system is the wear of the connecting elements, in particular the asymmetrical wearing of said elements. In order to avoid said asymmetrical wear and ensure a homogeneous wear of the connecting blades 20 of the connector assembly 14, the connecting configuration of the connection transmitter 10 enables to connect any guidewire 12 introduced inside the longitudinal connecting element 18, with each connection blade 20 at the same time. This is made possible by the transversal translation movement of the longitudinal connecting element 18 within the casing 16.

The wire longitudinal locking elements 24 cooperate together when the connection transmitter 10 is in its connecting configuration in order to avoid any longitudinal sliding of the introduced guidewire 12.

In the embodiment shown on the figures, the default, or resting configuration of the longitudinal connecting element 18 is the connecting configuration. In some not represented alternative embodiment, the default or resting configuration could be the non-connecting configuration. The default/resting configuration is maintained by means of a transversal locking element 28 secured to the longitudinal connecting element 18 and aimed at transversally cooperating with the casing 16. In the embodiment depicted on figures 7a, 7b, 7c, the transversal locking element 28 comprises a spring extending between the casing 16 and the longitudinal connecting element 28, thus continually pushing the connecting element 18 towards the casing 16, in a downwards way. The spring is axially locked by means of two pawns 28a, 28b (see figure 8). In those embodiments, in order to deactivate the transversal locking element 28, the spring has to be activated/compressed. Therefore, in those embodiments, when the spring is activated/compressed, the transversal locking element 28 is deactivated/unlocked and the connection transmitter 10 is in its non-connecting configuration. More precisely, when the locking element 28 is deactivated/unlocked, the connecting element 28 can be pushed upwards to the other half of the casing 16. In an alternative embodiment, when said spring is activated/compressed, the transversal locking element 28 is deactivated/unlocked and the connection transmitter 10 is in its connecting configuration.

To allow activation of the longitudinal connecting element 18, the longitudinal connecting element 18 comprises an activation switch 30, said activation switch 30 being operable from outside the casing. More precisely, the activation switch 30 enables to deactivate/unlock the transversal locking element 28 and thus enabling the longitudinal connecting element 18 to translate transversally (along the Y axis), thus leading to a configuration change. In the embodiment depicted on figures 5a and 5b, the activation switch 30 comprises a slider 32 which is aimed at being activated with a user's thumb. The slider 32 is secured to the connecting element 18. The slider 32 cooperates with the casing 16, particularly the downside of the casing 16 by means of sliding elements 34. The sliding elements 34 may comprise at least one tab 34a located on one of the slider 32 and the casing 16 (for instance at the opening 16a), and at least one corresponding transversal groove 34b located on the other one of the casing 16 and the slider 32, depending on the embodiment. Each tab 34a is aimed at sliding inside its corresponding transversal groove 34b, thus allowing the slider 32 to slide transversally with regards to the casing 16, and thus allowing the connected element 18 to slide transversally inside the casing 16.

The connection transmitter 10 according to the present invention enables to implement a connection process aimed at connecting a guidewire 12 to a connector assembly 14 of said connection transmitter 10. With regards to the represented embodiments, the connection process comprises following steps in the order of enunciation:
- switching of the connection transmitter 10 into its non-connecting configuration by means of the activation switch 30,
- longitudinally introducing a guidewire 12 inside the longitudinal cavity 22 of the connecting element 18,
- switching the connection transmitter 10 into its connecting configuration, in order to put the guidewire 12 in contact with at least one of the connection blades 20 of the connector assembly 14.

In some alternative embodiment in which the default/resting configuration is the non-connecting configuration, the first step would not exist.

## Claims

1. Connection transmitter (10) aimed at connecting a guidewire (12) to a connector assembly (14), the connection transmitter (10) comprising:
- a connector assembly (14),
- a casing (16) surrounding at least partially the connector assembly (14),
- a longitudinal connecting element (18) extending inside the casing (16) along a longitudinal axis (X),
**wherein** the connecting element (18) is translatable, within the casing (16), along a transversal axis (Y) perpendicular to the longitudinal axis (X), said longitudinal connecting element (18) being further aimed at receiving the guidewire (12),
**wherein** the connector assembly (14) comprises at least one connection blade (20) extending along the transversal axis (Y), and the connecting element (18) comprises at least one transversal cavity (26) aimed at receiving at least partially the guidewire (12), and further aimed at cooperating, by transversal translation, with the at least one connection blade (20),
**wherein** the connection transmitter (10) presents at least two configurations:
- a non-connecting configuration, in which the at least one transversal cavity (26) of the longitudinal connecting element (18) and the at least one connection blade (20) of the connector assembly (14) are transversely disengaged,
- a connecting configuration, in which the at least one transversal cavity (26) of the longitudinal connecting element (18) and the at least one connection blade (20) of the connector assembly (14) are transversely cooperating by engagement,
**wherein** the connecting configuration enables to connect the guidewire (12) introduced inside the longitudinal connecting element (18) with the at least connection blade (20) of the connector assembly (14).

2. Connection transmitter (10) according to the preceding claim, wherein the longitudinal connecting element (18) comprises a longitudinal cavity (22) extending along the longitudinal axis (X), said longitudinal cavity (22) being aimed at receiving the guidewire (12), said longitudinal cavity (22) intersecting each transversal cavity (26) of the longitudinal connecting element (18).

3. Connection transmitter (10) according to any one of the preceding claims, wherein the longitudinal connecting element (18) comprises at least one radial stop (23) configured to prevent radial or transversal motion of the guidewire (12) once said guidewire (12) is introduced in the longitudinal connecting element (18), only allowing the longitudinal sliding of said guidewire (12).

4. Connection transmitter (10) according to any one of the preceding claims, wherein the connecting configuration of the connection transmitter (10) is obtained by translating the longitudinal connecting element (18) along the transversal axis (Y), thus enabling to connect the guidewire (12) introduced inside the longitudinal connecting element (18) with each connection blade (20) of the connector assembly (14) at the same time.

5. Connection transmitter (10) according to any one of the preceding claims, wherein the number of connection blades (20) is equal to the number of transversal cavities (26) such that each connection blade (20) corresponds to a respective transversal cavity (26).

6. Connection transmitter (10) according to any one of the preceding claims, wherein the default configuration of the connection transmitter (10) is the connecting configuration, which is maintained by means of transversal locking element (28) aimed at transversally cooperating with the casing (16).

7. Connection transmitter (10) according to the preceding claim, wherein the transversal locking element (28) comprises a spring aimed at extending between the casing (16) and the longitudinal connecting element (18).

8. Connection transmitter (10) according to the preceding claim, wherein the longitudinal connecting element (18) and the casing (16) both comprise wire longitudinal locking elements (24) aimed at cooperating together when the connection transmitter (10) is in its connecting configuration in order to prevent longitudinal sliding of the introduced guidewire (12).

9. Connection transmitter (10) according to any one of the preceding claims, wherein the longitudinal connecting element (18) comprises an activation switch (30) to switch the longitudinal connecting element (18) between the connecting configuration and the non-connecting configuration, said activation switch (30) being operable from outside the casing (16).

10. Connection transmitter (10) according to the preceding claim, wherein the activation switch (30) comprises a slider aimed at being pushed with a user's thumb.

11. Connection kit comprising a connection transmitter (10) according to any one of the preceding claims, and a guidewire (12) aimed at being introduced at least partially inside the connection transmitter (10).

12. Connection kit according to the preceding claim, wherein the connector assembly (14) comprises N connection blades (20) extending along the transversal axis (Y), and wherein the longitudinal connecting element (18) comprises N transversal cavities (26), each transversal cavity (26) being aimed at cooperating, by transversal translation, with one corresponding connection blade (20) of the connector assembly (14), N being an integer equal to a number of contact zones on a proximal end of the guide wire.

13. Connection kit according to any one of the preceding claims, wherein a first end of the guidewire (12) comprises a series of contact zones (13) arranged along a longitudinal direction of the guidewire (12), each electrical contact being associated to a respective transversal cavity (26), a distance along the longitudinal axis (X) between two transversal cavities (26) being equal to a distance, along the longitudinal axis of the guidewire, between the two corresponding contact zones (13).

14. Connection process aimed at connecting a guidewire (12) to a connector assembly (14) of a connection transmitter (10), the connection process being implemented by means of a connection transmitter (10) according to any one of claims 1 to 13, said connection process comprising following steps in the order of enunciation:
- longitudinally introducing a first end of the guidewire (12) inside the longitudinal connecting element (18),
- switching the connection transmitter (10) into its connecting configuration, in order to put the guidewire (12) in contact with at least one of the connection blades (20) of the connector assembly (14).

15. Connection process according to the precedent claim, wherein the process comprises a step before the introduction of the guidewire (12) inside the longitudinal connecting element (18), said additional step being the switching of the connection transmitter (10) into its non-connecting configuration.

## Patentansprüche

1. Verbindungssender (10), der darauf ausgerichtet ist, einen Führungsdraht (12) mit einer Verbinderbaugruppe (14) zu verbinden, wobei der Verbindungssender (10) umfasst:
- eine Verbinderbaugruppe (14),
- ein Gehäuse (16), das die Verbinderbaugruppe (14) mindestens teilweise umgibt,
- ein Längsverbindungselement (18), das sich im Inneren des Gehäuses (16) entlang einer Längsachse (X) erstreckt,
**wobei** das Verbindungselement (18) innerhalb des Gehäuses (16) entlang einer Querachse (Y) senkrecht zur Längsachse (X) verschiebbar ist, wobei das Längsverbindungselement (18) weiter darauf ausgerichtet ist, den Führungsdraht (12) aufzunehmen,
**wobei** die Verbinderbaugruppe (14) mindestens ein Verbindungsblatt (20) umfasst, das sich entlang der Querachse (Y) erstreckt, und das Verbindungselement (18) mindestens einen Querhohlraum (26) umfasst, der darauf ausgerichtet ist, den Führungsdraht (12) mindestens teilweise aufzunehmen, und weiter darauf ausgerichtet ist, durch Querverschiebung mit dem mindestens einen Verbindungsblatt (20) zusammenzuwirken,
**wobei** der Verbindungssender (10) mindestens zwei Konfigurationen aufweist:
- eine Nicht-Verbindungskonfiguration, in der der mindestens eine Querhohlraum (26) des Längsverbindungselements (18) und das mindestens eine Verbindungsblatt (20) der Verbinderbaugruppe (14) in Querrichtung außer Eingriff stehen,
- eine Verbindungskonfiguration, in der der mindestens eine Querhohlraum (26) des Längsverbindungselements (18) und das mindestens eine Verbindungsblatt (20) der Verbinderbaugruppe (14) durch Eingriff in Querrichtung zusammenwirken,
**wobei** die Verbindungskonfiguration es ermöglicht, den in das Innere des Längsverbindungselements (18) eingeführten Führungsdraht (12) mit dem mindestens einen Verbindungsblatt (20) der Verbinderbaugruppe (14) zu verbinden.

2. Verbindungssender (10) nach dem vorstehenden Anspruch, wobei das Längsverbindungselement (18) einen Längshohlraum (22) umfasst, der sich entlang der Längsachse (X) erstreckt, wobei der Längshohlraum (22) darauf ausgerichtet ist, den Führungsdraht (12) aufzunehmen, wobei der Längshohlraum (22) jeden Querhohlraum (26) des Längsverbindungselements (18) schneidet.

3. Verbindungssender (10) nach einem der vorstehenden Ansprüche, wobei das Längsverbindungselement (18) mindestens einen radialen Anschlag (23) umfasst, der so konfiguriert ist, dass er radiale oder Querbewegung des Führungsdrahtes (12) verhindert, sobald der Führungsdraht (12) in das Längsverbindungselement (18) eingeführt ist, wobei nur das Längsgleiten des Führungsdrahtes (12) zugelassen wird.

4. Verbindungssender (10) nach einem der vorstehenden Ansprüche, wobei die Verbindungskonfiguration des Verbindungssenders (10) durch Verschieben des Längsverbindungselements (18) entlang der Querachse (Y) erhalten wird, wodurch es ermöglicht wird, den in das Innere des Längsverbindungselements (18) eingeführten Führungsdraht (12) mit jedem Verbindungsblatt (20) der Verbinderbaugruppe (14) gleichzeitig zu verbinden.

5. Verbindungssender (10) nach einem der vorstehenden Ansprüche, wobei die Anzahl von Verbindungsblättern (20) gleich der Anzahl von Querhohlräumen (26) ist, derart, dass jedes Verbindungsblatt (20) einem jeweiligen Querhohlraum (26) entspricht.

6. Verbindungssender (10) nach einem der vorstehenden Ansprüche, wobei die Standardkonfiguration des Verbindungssenders (10) die Verbindungskonfiguration ist, die mittels Querverriegelungselement (28), das darauf ausgerichtet ist, in Querrichtung mit dem Gehäuse (16) zusammenzuwirken, aufrechterhalten wird.

7. Verbindungssender (10) nach dem vorstehenden Anspruch, wobei das Querverriegelungselement (28) eine Feder umfasst, die darauf ausgerichtet ist, sich zwischen dem Gehäuse (16) und dem Längsverbindungselement (18) zu erstrecken.

8. Verbindungssender (10) nach dem vorstehenden Anspruch, wobei sowohl das Längsverbindungselement (18) als auch das Gehäuse (16) Draht-Längsverriegelungselemente (24) umfassen, die darauf ausgerichtet sind, zusammenzuwirken, wenn sich der Verbindungssender (10) in seiner Verbindungskonfiguration befindet, um Längsgleiten des eingeführten Führungsdrahtes (12) zu verhindern.

9. Verbindungssender (10) nach einem der vorstehenden Ansprüche, wobei das Längsverbindungselement (18) einen Aktivierungsschalter (30) umfasst, um das Längsverbindungselement (18) zwischen der Verbindungskonfiguration und der Nicht-Verbindungskonfiguration umzuschalten, wobei der Aktivierungsschalter (30) von außerhalb des Gehäuses (16) bedienbar ist.

10. Verbindungssender (10) nach dem vorstehenden Anspruch, wobei der Aktivierungsschalter (30) einen Schieber umfasst, der darauf ausgerichtet ist, mit dem Daumen eines Benutzers geschoben zu werden.

11. Verbindungsset, das einen Verbindungssender (10) nach einem der vorstehenden Ansprüche und einen Führungsdraht (12) umfasst, der darauf ausgerichtet ist, mindestens teilweise in das Innere des Verbindungssenders (10) eingeführt zu werden.

12. Verbindungsset nach dem vorstehenden Anspruch, wobei die Verbinderbaugruppe (14) N Verbindungsblätter (20) umfasst, die sich entlang der Querachse (Y) erstrecken, und wobei das Längsverbindungselement (18) N Querhohlräume (26) umfasst, wobei jeder Querhohlraum (26) darauf ausgerichtet ist, durch Querverschiebung mit einem entsprechenden Verbindungsblatt (20) der Verbinderbaugruppe (14) zusammenzuwirken, wobei N eine ganze Zahl ist, die gleich einer Anzahl von Kontaktzonen an einem proximalen Ende des Führungsdrahtes ist.

13. Verbindungsset nach einem der vorstehenden Ansprüche, wobei ein erstes Ende des Führungsdrahtes (12) eine Reihe von Kontaktzonen (13) umfasst, die entlang einer Längsrichtung des Führungsdrahtes (12) angeordnet sind, wobei jeder elektrische Kontakt einem jeweiligen Querhohlraum (26) zugeordnet ist, wobei ein Abstand entlang der Längsachse (X) zwischen zwei Querhohlräumen (26) gleich einem Abstand entlang der Längsachse des Führungsdrahtes zwischen den zwei entsprechenden Kontaktzonen (13) ist.

14. Verbindungsprozess, der darauf ausgerichtet ist, einen Führungsdraht (12) mit einer Verbinderbaugruppe (14) eines Verbindungssenders (10) zu verbinden, wobei der Verbindungsprozess mittels eines Verbindungssenders (10) nach einem der Ansprüche 1 bis 13 implementiert wird, wobei der Verbindungsprozess folgende Schritte in der Aufzählungsreihenfolge umfasst:
- Einführen eines ersten Endes des Führungsdrahtes (12) in Längsrichtung in das Innere des Längsverbindungselements (18),
- Umschalten des Verbindungssenders (10) in seine Verbindungskonfiguration, um den Führungsdraht (12) mit mindestens einem der Verbindungsblätter (20) der Verbinderbaugruppe (14) in Kontakt zu bringen.

15. Verbindungsprozess nach dem vorstehenden Anspruch, wobei der Prozess einen Schritt vor dem Einführen des Führungsdrahtes (12) in das Innere des Längsverbindungselements (18) umfasst, wobei der zusätzliche Schritt das Umschalten des Verbindungssenders (10) in seine Nicht-Verbindungskonfiguration ist.

## Revendications

1. Emetteur de transmission (10) destiné à relier un guide-fil (12) à un ensemble connecteur (14), le connecteur de transmission (10) comprenant :
- un ensemble connecteur (14),
- un boîtier (16) entourant au moins partiellement l'ensemble connecteur (14),
- un élément de liaison longitudinal (18) s'étendant à l'intérieur du boîtier (16) le long d'un axe longitudinal (X),
l'élément de liaison (18) étant déplaçable en translation à l'intérieur du boîtier (16) le long d'un axe transversal (Y) perpendiculaire à l'axe longitudinal (X), ledit élément de liaison longitudinal (18) étant en outre destiné à recevoir le guide-fil (12),
l'ensemble connecteur (14) comprenant au moins une lame de connexion (20) s'étendant le long de l'axe transversal (Y), et l'élément de liaison (18) comprenant au moins une cavité transversale (26) destinée à recevoir au moins partiellement le guide-fil (12), et destinée en outre à coopérer, par translation transversale, avec ladite lame de connexion (20),
l'émetteur de transmission (10) présentant au moins deux configurations :
- une configuration de non-connexion, dans laquelle ladite cavité transversale (26) de l'élément de liaison longitudinal (18) et ladite lame de connexion (20) de l'ensemble connecteur (14) sont désengagées transversalement,
- une configuration de connexion, dans laquelle ladite cavité transversale (26) de l'élément de liaison longitudinal (18) et ladite lame de connexion (20) de l'ensemble connecteur (14) coopèrent transversalement par engagement,
la configuration de connexion permettant de relier le guide-fil (12) introduit à l'intérieur de l'élément de liaison longitudinal (18) avec ladite lame de connexion (20) de l'ensemble connecteur (14).

2. Emetteur de connexion (10) selon la revendication précédente, dans lequel l'élément de liaison longitudinal (18) comprend une cavité longitudinale (22) s'étendant le long de l'axe longitudinal (X), ladite cavité longitudinale (22) étant destinée à recevoir le guide-fil (12), ladite cavité longitudinale (22) intersectant chaque cavité transversale (26) de l'élément de liaison longitudinal (18).

3. Émetteur de connexion (10) selon l'une quelconque des revendications précédentes, dans lequel l'élément de liaison longitudinal (18) comprend au moins un arrêt radial (23) configuré pour empêcher le mouvement radial ou transversal du guide-fil (12) une fois ledit guide-fil (12) introduit dans l'élément de liaison longitudinal (18), permettant uniquement le glissement longitudinal dudit guide-fil (12).

4. Émetteur de connexion (10) selon l'une quelconque des revendications précédentes, dans lequel la configuration de connexion de l'émetteur de connexion (10) est obtenue par la translation de l'élément de liaison longitudinal (18) le long de l'axe transversal (Y), permettant ainsi de connecter le guide-fil (12) introduit à l'intérieur de l'élément de liaison longitudinal (18) avec chaque lame de connexion (20) de l'ensemble connecteur (14) simultanément.

5. Émetteur de connexion (10) selon l'une quelconque des revendications précédentes, dans lequel le nombre de lames de connexion (20) est égal au nombre de cavités transversales (26) de telle sorte que chaque lame de connexion (20) correspond à une cavité transversale (26) respective.

6. Émetteur de connexion (10) selon l'une quelconque des revendications précédentes, dans lequel la configuration par défaut de l'émetteur de connexion (10) est la configuration de connexion, qui est maintenue au moyen d'un élément de verrouillage transversal (28) destiné à coopérer transversalement avec le boîtier (16).

7. Émetteur de connexion (10) selon la revendication précédente, dans lequel l'élément de verrouillage transversal (28) comprend un ressort destiné à s'étendre entre le boîtier (16) et l'élément de liaison longitudinal (18).

8. Émetteur de connexion (10) selon la revendication précédente, dans lequel l'élément de liaison longitudinal (18) et le boîtier (16) comprennent tous deux des éléments de verrouillage longitudinal de fil (24) destinés à coopérer ensemble lorsque l'émetteur de connexion (10) est dans sa configuration de connexion afin d'empêcher le glissement longitudinal du guide-fil (12) introduit.

9. Émetteur de connexion (10) selon l'une quelconque des revendications précédentes, dans lequel l'élément de liaison longitudinal (18) comprend un interrupteur d'activation (30) permettant de basculer l'élément de liaison longitudinal (18) entre la configuration de connexion et la configuration de non-connexion, ledit interrupteur d'activation (30) étant actionnable depuis l'extérieur du boîtier (16).

10. Émetteur de connexion (10) selon la revendication précédente, dans lequel l'interrupteur d'activation (30) comprend un curseur destiné à être poussé avec le pouce de l'utilisateur.

11. Kit de connexion comprenant un émetteur de connexion (10) selon l'une quelconque des revendications précédentes, et un guide-fil (12) destiné à être introduit au moins partiellement à l'intérieur de l'émetteur de connexion (10).

12. Kit de connexion selon la revendication précédente, dans lequel l'ensemble connecteur (14) comprend N lames de connexion (20) s'étendant le long de l'axe transversal (Y), et dans lequel l'élément de liaison longitudinal (18) comprend N cavités transversales (26), chaque cavité transversale (26) étant destinée à coopérer, par translation transversale, avec une lame de connexion correspondante (20) de l'ensemble connecteur (14), N étant un entier égal à un nombre de zones de contact sur une extrémité proximale du guide-fil.

13. Kit de connexion selon l'une quelconque des revendications précédentes, dans lequel une première extrémité du guide-fil (12) comprend une série de zones de contact (13) disposées le long d'une direction longitudinale du guide-fil (12), chaque contact électrique étant associé à une cavité transversale respective (26), une distance le long de l'axe longitudinal (X) entre deux cavités transversales (26) étant égale à une distance, le long de l'axe longitudinal du guide-fil, entre les deux zones de contact correspondantes (13).

14. Processus de connexion visant à connecter un guide-fil (12) à un ensemble connecteur (14) d'un émetteur de connexion (10), le processus de connexion étant mis en oeuvre au moyen d'un émetteur de connexion (10) selon l'une quelconque des revendications 1 à 13, ledit processus de connexion comprenant les étapes suivantes dans l'ordre d'énonciation :
- introduire longitudinalement une première extrémité du guide-fil (12) à l'intérieur de l'élément de liaison longitudinal (18),
- basculer l'émetteur de connexion (10) dans sa configuration de connexion, afin de mettre le guide-fil (12) en contact avec au moins une des lames de connexion (20) de l'ensemble connecteur (14).

15. Processus de connexion selon la revendication précédente, dans lequel le processus comprend une étape avant l'introduction du guide-fil (12) à l'intérieur de l'élément de liaison longitudinal (18), ladite étape supplémentaire étant la bascule de l'émetteur de connexion (10) dans sa configuration de non-connexion.
